# EUROPÄISCHE PATENTSCHRIFT

(11) **EP 0 350 723 B1**
(45) Veröffentlichungstag und Bekanntmachung des Hinweises auf die Patenterteilung: **10.08.1994**
(21) Anmeldenummer: 89111845.7
(22) Anmeldetag: 29.06.1989
(51) Int. Cl.: B01L 3/00, A61L 2/26

(54) **Ventil zum Anschliessen von Objekten an vor Ort zu sterilisierenden Behältern oder Leitungen**
Valve for connecting objects to container or ducts to be sterilised in situ
Vanne de connection d'objets à des récipients ou tubulures à stériliser sur place

(30) Priorität: 13.07.1988 DE 3823711
(43) Veröffentlichungstag der Anmeldung: 17.01.1990
(73) Patentinhaber: B. BRAUN BIOTECH INTERNATIONAL GmbH, 34212 Melsungen (DE)
(72) Erfinder: Hediger, Jacob, CH-8633 Wolfhausen (CH)
(74) Vertreter: Vonnemann, Gerhard, Dr.-Ing.

(56) Entgegenhaltungen:
- EP-A- 0 295 408
- DE-A- 2 609 825
- US-A- 4 665 944
- SOVIET INVENTIONS ILLUSTRATED, Woche B05, 14. März 1979, Nr. D16 J04 R 16,09672B/05, Derwent Publications Ltd, Londen, GB; & SU-A-597 938 (V.E. MATVEEV) 27-02-1978

## Beschreibung

Die Erfindung betrifft ein sterilisierbares Objekt, wie Behälter oder Leitung, mit Anschlußventil, das eine äußere Anschlußöffnung, ein an einer Anschlußöffnung des Objekts dichtend anbringbares Ventilgehäuse mit zum Innenraum des Objekts führender Sitzöffnung, eine in dieser zwischen einer Sperrstellung und einer Durchtrittsstellung radial abgedichtet verschiebbar geführte Ventilspindel aufweist.

Behälter, z.B. Bioreaktoren, in denen unter sterilen Bedingungen Stoffe gelagert und/oder in Arbeitsprozessen verändert werden, müssen zur Probenahme, zum Hinzu-dosieren verschiedener Reagenzien sowie zum Abernten Anschlußöffnungen aufweisen. Die anzuschließenden Objekte müssen dabei ebenfalls unter sterilen Bedingungen montiert werden. Weit verbreitet ist ein Verfahren, bei dem auf einem speziellen Anstechstutzen des Behälters ein dicht eingespannter Elastomerformling als Membran den sterilisierten Behälter verschließt. Mit einer ebenfalls sterilisierten spitzen Hohlnadel wird diese Membran von Hand durchstochen und so eine Sterilverbindung zwischen Innen und Außen herstellt. Damit bei und nach dem Entnehmen der Anstechnadel aus dem Autoklaven mit angeschlossenem Zubehör wie z.B. Schlauchleitung keine Kontamination der Nadel mit Mikroorganismen aus der unsterilen Umgebung stattfinden kann, wird diese Nadel vor dem Sterilisieren mit einer geeigneten Folie umwickelt bzw. in diese eingepackt und dicht verklebt. Den gleichen Zweck erreicht man durch eine Hülse mit eingebautem Mikrofilter, die über die Nadel geschoben wird und auch unter dem Namen "Sterilhülse" bekannt ist. Folie bzw. Sterilhülse müssen unmittelbar vor dem Anstechen von Hand entfernt werden. Um eine Kontamination des anzustechenden Objektes zu vermeiden, müssen Membran und Anstechnadel nach dem Auspacken bis zum Anstechen mit Flammen steril gehalten werden. Dies ist die risikoreichste Phase der bekannten Arbeitsweise.

Zur Verringerung dieses Risikos werden, wenn es um das Hinzudosieren von Reagenzien geht, Dosierventile eingesetzt bzw. die erforderlichen Anschlußleitungen fest installiert und zusätzlich auch die Anschlußleitung unter sterilen Bedingungen gehalten, wobei aufwendige Leitungsinstallationen mit entsprechenden Ventilen vorgesehen werden müssen, damit die peripheren Anschlußleitungen sterilisiert werden können. Dazu gehören meist Dampf- und Kondensatanschlüsse und aufwendige Steuerungseinrichtungen, damit diese Ventile dann in genau festgelegten Sequenzen bedient bzw. angesteuert werden. Nur auf diese Weise ist die geforderte Sicherheit zu gewährleisten.

Nachteilig ist, daß besonders in Labors, der dazu notwendige Dampf nicht ohne weiteres zur Verfügung steht und deshalb zusätzliche Investitionen für die entsprechende Infrastruktur notwendig werden. Wird die Verbindung zum Behälter mittels einer Hohlnadel hergestellt, indem diese durch die Membran hindurchgestoßen wird, so muß man dabei das erhöhte Risiko einer Kontamination des Behälterinhaltes und der Umgebung in Kauf nehmen. Besonders risikoreich ist dabei ein Anstechen von Behältern im Bereich unterhalb des Flüssigkeitsspiegels, also direkt in die Flüssigkeit. In explosionsgefährdeten Bereichen ist das Durchstechen der Membran überhaupt nicht anwendbar. Wenn beispielsweise ein Fermenter einmal angestochen wurde, kann dieser nicht mehr nachsterilisiert werden, da die perforierte Membran und der Fermenter dann nicht mehr sterilisiert werden können. Außerdem ist nachteilig, daR für pathogene Anwendungen, ein Durchstechen der Membran zu risikoreich ist.

Die nachteiligen Folgen einer Kontamination, die bei den bekannten Verfahren nicht auszuschließen sind, sind in der Regel schwerwiegend und liegen beispielsweise darin begründet, daß Mensch, Tier und Pflanze in der Umgebung gefährdet werden. Außerdem führt die Kontamination von Prozeßchargen zu höheren Kosten durch die verlorene Charge und deren umweltgerechter Beseitigung. Die Produktionsziele werden dann nicht erreicht. Es müssen zusätzliche Anlagen als Reserve bereitgestellt werden. Dies hat natürlich wiederum erhöhte Investitionskosten zur Folge.

In der EP 295 408 A2 wird ein Ventil beschrieben, das durch eine Außenwand eines Bioreaktorgefäßes hindurchführt. Mit dem Ventil wird eine Dampfsterilisation des Innenraumes des Reaktorgefäßes und der unter dem Flüssigkeitsspiegel des Reaktorgefäßes ragenden Rohre ermöglicht. Das Ventil weist eine mit einer axial durchgehenden Bohrung versehene Ventilspindel auf. Nachteilig ist, daß das Ventil ein hermetisches Verschließen des Behälters nicht ermöglicht, da es lediglich dazu geeignet ist, wahlweise eine Verbindung zum Reaktorgefäß oder zu einem im Reaktorgefäß angeordneten Tauchrohr herzustellen. Das Ventil wird mit einem nachfolgend angeordneten Drei/Zwei-Wegeventil verwendet.

Aus der SU 597 938 ist eine Vorrichtung zur sterilen Probennahme bei mikrobiologischen Verfahren bekannt, die ein Dampfsterilisationssystem mit Kondensator und hermetisch abgedichtetes mit einem Anschlußventil versehenes Probennahmegefäß aufweist. Nach der Sterilisation in einem Autoklaven werden das Probengefäß und das Ventil mit einer Zwischenkammer verbunden. Das Probengefäß mit Ventil kann an der Zwischenkammer gelöst werden und zur Weiterbehandlung der Probe abtransportiert werden.

Aus der US 4,665,944 ist ein Hochdruckventil mit einer radial abgedichteten verschiebbar geführten Ventilspindel bekannt, das zwischen einer Entleerungs- und Durchtrittsstellung schaltbar ist. Die axial mit einer Sackbohrung versehene Ventilspindel weist zwei deren Umfangsflächen mündende Seitenöffnungen auf. Ferner sind im Ventilgehäuse eine an die Umfangsfläche der Ventilspindel angrenzende Ventilkammer und eine Entleerungskammer angeordnet. Nachteilig ist, daß das Ventil lediglich zur Entspannung unter Hochdruck stehender Flüssigkeiten dient, wobei beide Ventilanschlußleitungen in der Entleerungsstellung gegen Umgebungsdruck entspannt werden.

Aus der DE-A-2.609.825, von der die Erfindung ausgeht, ist ein sterilisierbares Objekt, wie Behälter oder Leitung, mit Anschlußventil bekannt, das eine äußere Anschlußöffnung, ein an einer Anschlußöffnung des Objekts dichtend anbringbares Ventilgehäuse mit zum Innenraum des Objekts führender Sitzöffnung, eine in dieser zwischen einer Sperrstellung und einer Durchtrittsstellung radial abdichtend verschiebbar geführte Ventilspindel aufweist. Dabei dichtet ein Ventilteller mit radialer Dichtung in der Schließstellung die Ablauf- bzw. Zufuhröffnung ab.

Sie ist ungeeignet, eine Verbindung zu einem Tauchrohr herzustellen.

Aufgabe der Erfindung ist es, eine andere Ventilbauform anzugeben, die für Anwendungsfälle in der Biotechnologie über besonders wenige tote Ecke verfügt, damit das Kontaminationsrisiko vermindert wird.

Diese Aufgabe wird durch jede der Merkmalskombinationen der nebengeordneten Ansprüche 1 bis 3 gelöst.

Durch einfache Zusatzausrüstungen können diese Ventilbauformen gleichzeitig als Abschlußventil und als Umschaltventil zur Verbindung mit einem Tauchrohr dienen. Sie sind damit besonders universell für die belange der Biotechnologie einsetzbar. Durch diese universelle Einsetzbarkeit kann die Anzahl der Ventile an Bioreaktoren vermindert werden, wodurch nicht nur Kostenvorteile erzielt werden, sondern auch das Kontaminationsrisiko wertvoller Chargen vermindert wird.

Vorteilhaft können somit auch bestehende Anlagen nachgerüstet werden, ohne daß aufwendige Leitungsführungen und Steuereinrichtungen für ein zusätzliches Sterilisationsmedium vorzusehen wären.

Es ist möglich, das Ventil zunächst unter unsterilen Bedingungen zu montieren. Im geschlossenen Zustand des Ventils wird das anzuschließende Objekt sterilisiert, d.h. erst, nachdem die speziellen Anschlußventile in geschlossenem Zustand an diesem fest und dicht montiert worden sind. Dadurch werden die noch unsterilen wesentlichen Ventilpartien mit dem Objekt selbst sterilisiert. Es ist unerheblich, ob die angeschlossenen Ventile unter oder über dem Flüssigkeitsspiegel liegen. Erst nach der vor Ort erfolgten Sterilisation der angeschlossenen Objekte werden die Anschlußventile geöffnet und in Betrieb genommen. Dadurch ist der Einsatz dieser Ventile auch in explosionsgefährdeten Bereichen möglich.

Die Ventile lassen sich hinsichtlich ihrer Öffnungsrichtung unterscheiden. Außerdem bestehen wesentliche Unterschiede darin, ob eine Ventilkammer vorgesehen ist oder nicht. Die Anschlußöffnung kann entweder durch einen Stößel geführt sein oder aber direkt mit einer Ventilkammer verbunden sein.

In weiterer Ausgestaltung der Erfindung ist vorgesehen, daß das Anschlußventil eine Verbindung zu einem Tauchrohr aufweist. Damit ist es möglich, daß das von dem Behälterinnenraum abgewandte Ende der Ventilspindel innerhalb eines Tauchrohres beweglich angeordnet ist und gegenüber diesem mindestens in Durchtrittsstellung abgedichtet ist. Dadurch läßt sich das Ventil auch besonders günstig ohne großen konstruktiven Aufwand an Tauchrohre anschließen. Die Abdichtung sorgt dafür, daß die Ventile zu Entnahmezwecken nicht unterhalb des Flüssigkeitsspiegels angeordnet werden müssen, da die Dichtung auch gegen Unterdruck abdichtet. Alternativ ist es möglich, daß das Ventilgehäuse als ein in den Behälter ragender rohrförmiger Fortsatz mit seitlichen Öffnungen ausgebildet ist, der am Ende eine Kupplung zur Montage eines Tauchrohres aufweist. Dies ermöglicht eine besonders einfache Befestigung des Tauchrohres direkt am Ventil selbst. Zusätzliche Befestigungsorgane innerhalb des Behälters entfallen dadurch vorteilhaft. Durch die zusätzlichen Bohrungen innerhalb des Ventilgehäuses wird auch eine einwandfreie Sterilisation von schwer zugänglichen Stellen des Ventils bis zum Ventilsitz beim Sterilisieren des Behälters vor Ort erreicht. Ebenso ist es möglich, daß das Tauchrohr oder die Tauchleitung mit der Ventilspindel verbunden ist, wobei die Verbindung vorzugsweise mittels Überwurfmutter und Quetschdichtung erfolgt. Diese Befestigungsweise ermöglicht besonders kostengünstige Tauchrohrkonstruktionen, da Befestigungselemente innerhalb des sterilen Behälters vorteilhaft entfallen können.

In weiterer Ausgestaltung der Erfindung ist vorgesehen, daß das vom Behälterinnenraum abgewandte Ende der Ventilspindel an mindestens zwei Stellen gegenüber dem Ventilgehäuse abgedichtet ist, so daß die Dichtungen einen Sperraum um die Spindel bilden, in den ein oder mehrere Bohrungen mündend angeordnet sind. Für besonders hohe Anforderungen bei risikoreichen Prozessen läßt sich die Sicherheit vorteilhaft dadurch erhöhen, indem durch den Sperraum auch während des Prozesses ein sterilisierendes Medium hindurchgeleitet wird und/oder mit Druck überlagert wird.

In weiterer Ausgestaltung der Erfindung ist vorgesehen, daß das Anschlußventil eine Verbindung zu einem in Richtung des Behälters öffnenden Rückschlagventil aufweist. Besonders wenig tote Ecken, die eine sichere Sterilisation gefährden könnten, ergeben sich wenn die Ventilspindel und oder das Tauchrohr am zum Behälterinnenraum gerichteten Ende nur seitliche Öffnungen der Durchgangsbohrung aufweisen, die vorzugsweise von einer elastischen Membran, insbesondere von einem elastischen Schlauch, verschlossen sind. Dadurch wird auf vorteilhaft einfache und kostengünstige Weise ein Rückschlagventil verwirklicht. Beim Zudosieren von Medien in den Behälter kann es erfahrungsgemäß vorkommen, daß ein Leck im Druckbereich der Zudosierleitung entsteht, wie es beispielsweise beim Durchscheuern des Schlauches einer Schlauchpumpe geschehen kann. Der Zufuhrdruck fällt dann zusammen. Da üblicherweise der Behälter mit Überdruck betrieben wird, führt dies zu einem Zurückschlagen von Prozeßflüssigkeit oder Prozeßabgas, je nachdem, an welchem Ort das Ventil angeordnet ist. Die damit verbundenen Folgen werden vorteilhaft durch das Rückschlagventil vermieden.

Auch die Maßnahme, daß das Ventilgehäuse innerhalb eines Einbauteils angeordnet ist, das vorzugsweise rohrförmig ausgebildet ist und an einem Ende einen äußeren Bund, und am anderen Ende ein Außengewinde für eine Überwurfmutter oder -schraube aufweist, dient einer einfacheren Montage. Im Falle einer Überwurfschraube weist das Einbauteil eine Öffnung mit einem Innengewinde mit Bund auf, in das die Schraube mit dem Ventil eingeschraubt wird. Dabei wird ein am Ventil angeformter Bund von der Schraube gegen den Bund der Öffnung gespannt.

In weiterer Ausgestaltung der Erfindung ist vorgesehen, daß die Ventilspindel, das Ventilgehäuse und das Einbauteil die Ventilkammer bildend angeordnet sind. Dadurch wird eine fertigungstechnisch besonders günstige Bauweise erzielt.

In weiterer Ausgestaltung der Erfindung ist vorgesehen, daß die Dichtung zwischen Ventilspindel und Ventilgehäuse auf der dem Behälterinnenraum abgewandten Seite eine Membrandichtung ist. Diese Dichtungsart weist den Vorteil auf, daß die Dichtstellen ortsunveränderlich sind. Anders ausgedrückt: es gibt gegen die unsterile Umgebung keine Dichtungen, die auf Dichtflächen gleiten. Dadurch können Leckverluste verringert werden. Dies ist besonders vorteilhaft, wenn während eines Arbeitsprozesses das Ventil mehrmalig betätigt wird. Dies gilt insbesondere, wenn die Membrandichtung ein Faltenbalg oder ein elastischer Schlauch ist.

Die Erfindung wird nachstehend in Form bevorzugter Ausführungsbeispiele unter Bezugnahme auf beiliegende Zeichnungen beschrieben, wobei gleichartige Teile in den verschiedenen Zeichnungsfiguren mit denselben Bezugszeichen versehen sind.

Die Figuren zeigen:
- Fig. 1: einen Schnitt durch das erfindungsgemäße Anschlußventil mit doppelter Abdichtung der Ventilspindel,
- Fig. 2: einen Schnitt durch das erfindungsgemäße Ventil mit einem relativ zum Behälter feststehenden Tauchrohr, das am Behälterstutzen (und nicht am Ventil) montiert ist,
- Fig. 3: einen Schnitt durch das erfindungsgemäße Ventil mit einem relativ zum Behälter beweglichen Tauchrohr,
- Fig. 4: einen Schnitt durch das erfindungsgemäße Ventil in Sperrstellung als Inline-Ausführung
- Fig. 5: einen Schnitt durch das erfindungsgemäße Ventil in Sperrstellung als Inline-Ausführung, jedoch ohne Ventilkammer,
- Fig. 6: einen Schnitt durch das erfindungsgemäße Ventil in Sperrstellung als Eckventil mit entgegengesetzter Betätigungsrichtung,
- Fig. 7: einen Schnitt durch ein selbsttätig öffendes Ventil gemäß der Erfindung und
- Fig. 8: einen Schnitt durch eine Ventilvariate mit Ventilkammer.

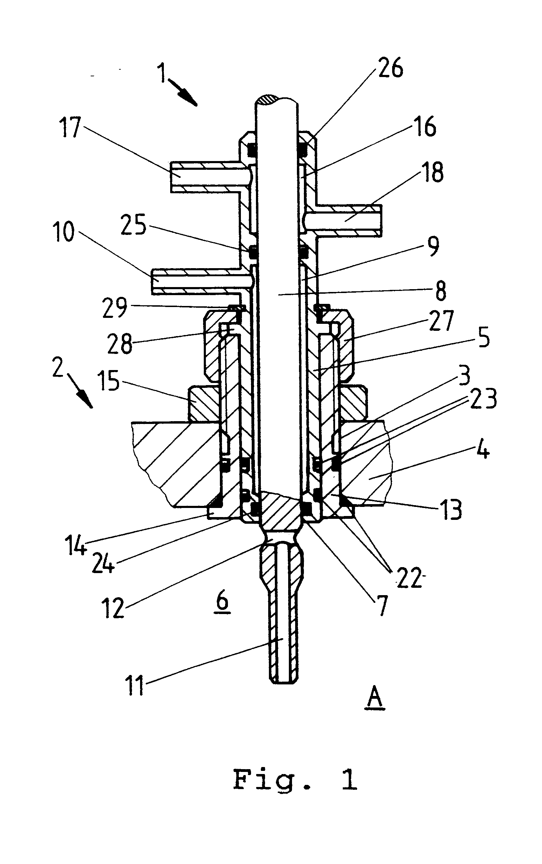

In Fig. 1 ist mit 1 das erfindungsgemäße Anschlußventilbezeichnet und mit 2 ein sterilisierbarer Behälter. Der sterilisierbare Behälter 2 weist Öffnungen 3 in seiner Behälterwandung 4 auf.

Das Ventil 1 hat ein Ventilgehäuse 5, das in der Einbaustellung zum Innenraum 6 des Behälters in eine Sitzöffnung 7 mündet, in der die Ventilspindel 8 axial verschieblich angeordnet ist. Von Ventilgehäuse 5 und Ventilspindel 8 wird die Ventilkammer 9 gebildet, in die eine äußere Anschlußöffnung 10 mündet. Der Ventilstößel 8 weist in seinem unteren Teil eine Durchgangsbohrung 11 auf, die zwei seitliche Öffnungen 12 und die Öffnung in der Stirnfläche der Ventilspindel 8 verbindet.

Zur Montage des Ventils ist zwischen Behälter 2 und Ventil 1 ein Einbauteil 13 angeordnet, das vom Behälterinnenraum 6 in die Anschlußöffnung 3 bis zum Anschlag 14 eingesteckt ist. Mittels Mutter 15, die auf einem Gewinde des Einbauteils 13 geschraubt ist, wird das Einbauteil 13 mit der Behälterwandung 4 verspannt

Oberhalb der Ventilkammer 9 ist des weiteren eine ringförmig um den Ventilstößel 8 angeordnete Sperrkammer 16 mit einer Einlaßöffnung 17 und einer Auslaßöffnung 18 angeordnet.

Die Dichtungen 22 dichten den Behälter und das Ventilgehäuse gegen den Behälterinnenraum ab. Die Dichtungen 23 dienen der Abdichtung der Spülöffnung bzw. des Durchbruchs und der Bohrung in der Behälterwandung. Die Dichtung 24 dichtet die Ventilkammer 9 zum Behälterinnenraum 6 ab; während die Dichtung 25 den Ventilraum 9 nach außen abdichtet. Zusätzlich ist die Dichtung 26 vorgesehen, so daß durch die Dichtungen 26 und 25 die Sperrkammer sowohl nach außen als auch zur Ventilkammer 9 abgedichtet ist.

Das Ventil ist in eine zylindrische Bohrung des Einbauteils 13 eingesteckt und mit diesem mittels der Überwurfmutter 27, die das gleiche Gewinde wie die Mutter 15 aufweist, verspannt. Die Überwurfmutter 27 ist dabei zwischen dem Bund 28 des Ventilgehäuses und dem Sicherungsring 29 drehbeweglich gehalten.

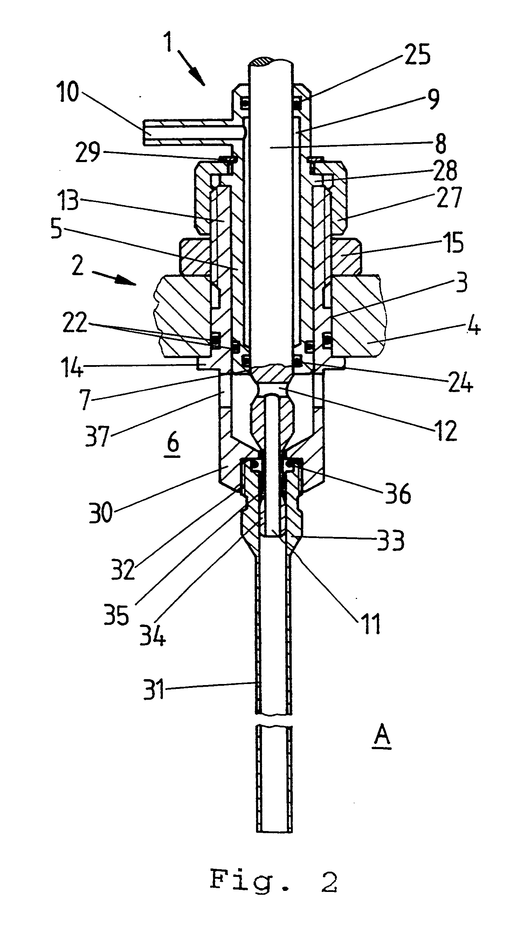

Fig. 2 zeigt das erfindungsgemäße Anschlußventil als Schnitt mit einer zusätzliche Halterung 30 am Einbauteil 13 zur Befestigung eines Tauchrohres 31. Dazu ist das Einbauteil 13 zum Behälterinnenraum 6 rohrförmig verlängert und an seinem Ende mit einem Gewinde 32 versehen, in das Einsteckende 33 des Tauchrohres 31 eingeschraubt werden kann.

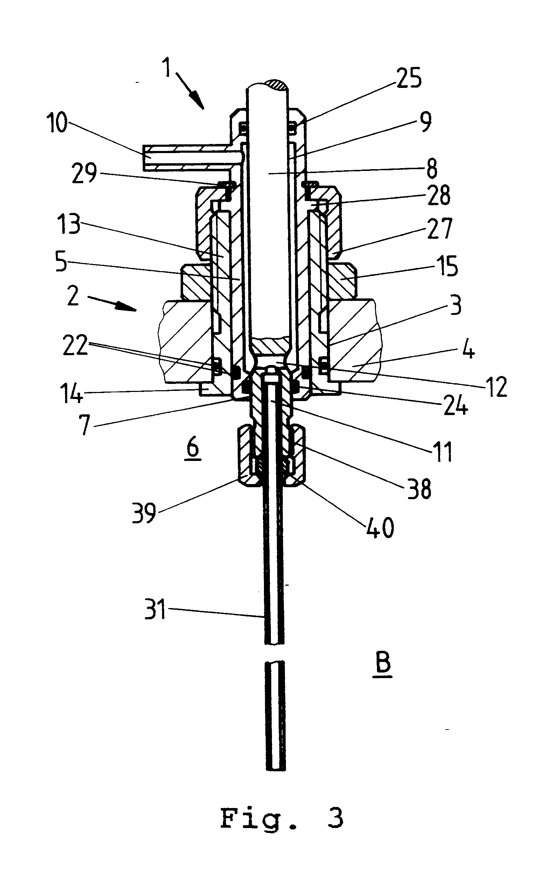

Die Ventilspindel 8 weist an ihrem zum Behälterinnenraum 6 gerichteten Ende einen Abschnitt 34 mit einem größeren und Abschnitt 35 mit einem kleineren Durchmesser auf. In der geschlossenen Stellung A befinden sich beide Abschnitte des Ventilstößelendes innerhalb des Tauchrohres 31. In der nicht dargestellten Durchlaßstellung B (Fig 3) dagegen, befindet sich der Abschnitt 35 des Ventilstoßelendes außerhalb des Tauchrohres, wobei die Dichtung 36 dann zwischen Abschnitt 34 und dem Tauchrohr angeordnet ist. Zum Zwecke des Sterilisierens hat die Tauchrohrhalterung Öffnungen 37, so daß der Raum zwischen Halterung und Ventilstößel in der Sperrstellung A von sterilisierenden Medien erreichbar ist.

Fig. 3 zeigt eine andersgeartete Ausgestaltung der Tauchrohrbefestigung. Das Tauchrohr 31 ist dabei in die Durchtrittsöffnung 11 des Ventilstößels 8 eingesteckt. Der Ventilstößel 8 weist an seinem zum Innenraum 6 des Behälters gerichteten Ende ein Gewinde 38 für die Überwurfmutter 39 auf. Zwischen dem Ende des Ventilstößels und der Überwurfmutter 39 ist eine Quetschdichtung 40 eingefügt. Durch Anziehen der Überwurfmutter 39 wird die Dichtung 40 zwischen dem Ende des Ventilstößels und der Überwurfmutter 39 komprimiert, so daß sie sich auf dem Tauchrohr 31 abstützt und dabei das Tauchrohr fest mit dem Ventilstößel 8 dichtend verbindet.

Fig. 4 zeigt einen Schnitt durch das erfindungsgemäße Ventil als Inline-Ausführung. Diese Ausführung unterscheidet sich von den oben beschriebenen eckventilartigen Ausführungen dadurch, daß hier der Anschluß über die Anschlußöffnung 10 koaxial mit der Ventilspindel 8 erfolgt. Dazu weist die Ventilspindel 8 eine koaxiale geführte Bohrung auf, die innerhalb der Ventilkammer 9 in zwei seitliche Austrittsöffnungen mündet. Der Pfeil zeigt die Richtung an, in die die Ventilspindel zum Öffnen bewegt wird.

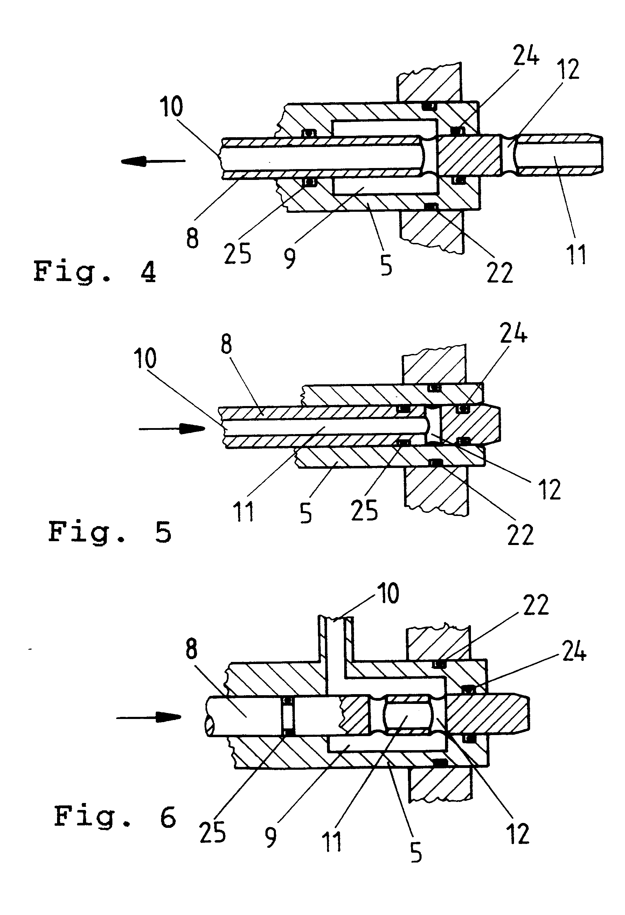

Figur 5 zeigt einen Schnitt durch eine weitere Inline-Ausführung auf, die im Unterschied zu den vorher beschriebenen Ausführungsformen ohne Ventilkammer ausgebildet ist. Dadurch ergibt sich ein vorteilhaft geringes Ventilvolumen, das leichter sterilisiert werden kann. Die Ventilspindel weist in dieser Ausführungsform lediglich eine einzige Durchgangsbohrung 11 auf, die in zwei seitliche Öffnungen 12 mündet. In Sperrstellung sind die Öffnungen 12 vom Ventilgehäuse 5 verschlossen. Die seitlich der Öffnungen 12 dichtenden Ringe 24 und 25 sind bei dieser Ausführungsform nicht wie in den oben beschriebenen Fällen innerhalb des Ventilgehäuses, sondern auf der Ventilspindel angeordnet. Zum Öffnen des Ventils wird die Ventilspindel in Pfeilrichtung zum Behälterinneren verschoben. Diese Ausführungsform läßt sich auch mit einem vorteilhaft geringen technischen Fertigungsaufwand herstellen.

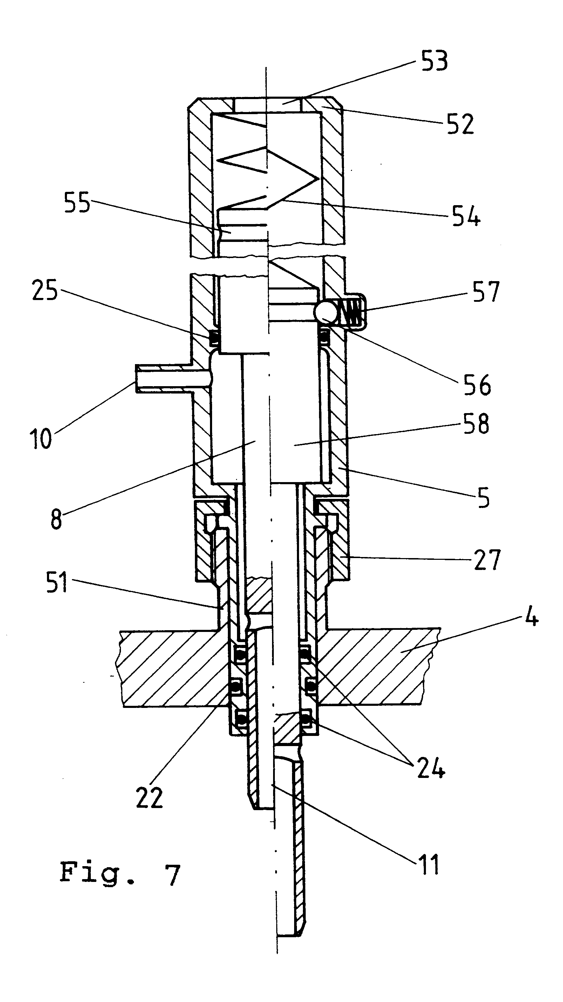

Figur 6 zeigt einen Schnitt durch eine andere Ausführungsform des erfindungsgemäßen Ventils, das als Eckventil ausgebildet ist, aber wie in Figur 6 zum Öffnen in Pfeilrichtung zum Behälterinneren in Pfeilrichtung bewegt wird. Dazu weist die Ventilspindel 8 in einem Teilbereich eine Durchgangsbohrung 11 auf, die beidseitig in seitlichen Öffnungen 12 innerhalb der Ventilkammer 9 mündet. Zur Umgebung hin dichtet der Ring 25 ab, der auf der Ventilspindel 8 innerhalb des Ventilgehäuses 5 angeordnet ist. Zum Behälter hin wird die Ventilkammer durch einen innerhalb des Gehäuses angeordneten Ring 24 abgedichtet.

Figur 7 zeigt eine Ventilausführung, die sich selbsttätig öffnet, sobald unter Druck ein Medium zugeführt wird. Links der strichpunktierten Linie ist das Ventil in Durchgangsstellung gezeichnet, rechts der Linie in Sperrstellung. Das Ventilgehäuse 5 ist mittels Überwurfmutter 27 auf einem mit Außengewinde versehenen Anschlußstutzen 51 des Behälters befestigt. Der Ventilstößel 8 ist bei dieser als Eckventil ausgebildeten Ausführung an seinem, dem Behälter abgewandten Ende, als Kolben 58 geformt. Auf der Mantelfläche des Kolbens 58 befindet sich eine Rastnut 55, in die die Kugel 56 durch Feder 57 in Sperrstellung einrastet. Zwischen dem oberen Ende des Kolbens 58 und dem als Widerlager 52 ausgebildeten oberen Ende des Ventilgehäuses 5 ist eine Feder 54 angeordnet, die den Kolben 58 mit Ventilstößel 8 selbsttätig in die rechts der strichpunktierten Linie dargestellte Sperrstellung drückt, sobald kein Druck in der Ventilkammer ansteht. Damit der Kolben 58 bei seinem Hub innerhalb des Ventilgehäuses keinen Gegendruck aufbaut, wird das Ventilgehäuse 5 durch die Öffnung 53 belüftet.

Das Ventil wird betätigt, indem man durch die Anschlußöffnung 10 ein Medium unter Druck zuführt. Durch die größere Fläche des kolbenartig ausgebildeten Endes 58 der Ventilspindel 8 wird die Ventilspindel aus der rechts von strichpunktierten Linie dargestellten Lage in die links von der Linie dargestellte Lage bewegt. Dabei drückt sie die Kugel 56 gegen die Kraft der Feder 57 aus der Rastnut 55 heraus. Sobald die Durchgangsbohrung 11 mit der Ventilkammer kommuniziert, verringert sich der in der Ventilkammer aufgebaute Druck so weit, daß der Kolben 58 in Gleichgewichtslage zum Stillstand kommt.

Wird der Fluß des zugeführten Mediums unterbrochen, so sinkt der Druck innerhalb der Ventilkammer so weit, daß die Feder 54 den Kolben 58 mit Ventilspindel 8 wieder in die Schließstellung 11 drückt, wobei die Kugel 56 durch die Kraft der Feder 57 wieder in die Rastnut 55 einrastet.

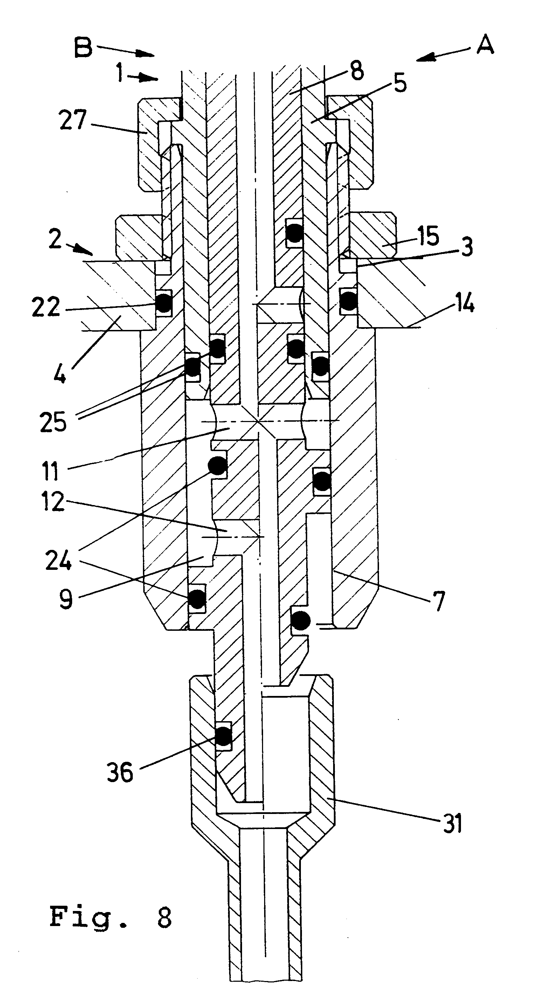

Fig. 8 zeigt eine Ventilvariante, bei der die Ventilkammer 9, anders als bei den zuvor beschriebenen Varianten, nach außen durch das Einbauteil 13, oben von der Stirnfläche des Ventilgehäuses 5 und unten durch einen Bund am Ventilstößel 8 gebildet wird. Links der strichpunktierten Mittellinie ist die Durchgangstellung des Ventils gezeigt, während rechts der strichpunktierten Mittellinie das Ventil in Sperrstellung dargestellt ist. Alle Dichtungen sind als außen liegende O-Ringe ausgebildet, so daß das Ventil sich besonders kostengünstig auch für kleine Durchmesser herstellen läßt.

Die erfindungsgemäßen Ventile funktionieren wie folgt:
In der in Fig. 1 dargestellten Stellung A sperrt das Ventil den Behälterinnenraum 6 hermetisch gegen außen ab. In dieser Stellung wird der Behälterinnenraum vor Ort sterilisiert. Die Durchgangsbohrung 11 und die seitlichen Öffnungen 12 befinden sich dabei innerhalb des Behälterinnenraums. Durch Betätigung der Ventilspindel 8 aus dem Behälterinnenraum heraus, werden die seitlichen Bohrungen 12 so weit bewegt, bis sie sich innerhalb der Ventilkammer 9 befinden. Über die seitlichen Öffnungen 12 und die Durchgangsbohrung 11 kommuniziert dann die Ventilkammer 9 mit dem Behälterinnenraum 6. In dieser Stellung kann über die Anschlußöffnung 10 ein Stoff zu einer im Behälter befindlichen Charge hinzudosiert werden, oder, je nach Einbauort des Ventils, auch Stoffe aus dem Behälterinnenraum abgezogen werden. Für spezielle Einsatzfälle kann auch eine Sperrkammer 16 vorgesehen werden, durch die über die Öffnungen 17, 18 ständig ein sterilisierendes Medium vorzugsweise unter Druck hindurchgeleitet oder vorgelegt wird. Kontaminationen der Außenwelt können damit sicher verhindert werden.

In Fig. 2 können in gesperrter Stellung A alle innerhalb des Behälters befindlichen Teile mit diesem gleichzeitig sterilisiert werden. Erst nach Bewegen der Ventilspindel 8 in die Durchtrittsstellung B (Fig. 3) gelangt dabei der mit dem größeren Durchmesser versehene Abschnitt 34 des Ventilstößels in den Bereich der Dichtung 36, die dann den Zwischenraum zwischen Ventilstößel und Tauchrohr 33 abdichtet. Dadurch ist es in dieser Stellung möglich, über das Tauchrohr 31 Medien an den gewünschten Stellen abzusaugen oder gezielt einzubringen. Dazu muß sich das untere Ende des Tauchrohrs 31 unterhalb der Flüssigkeitsoberfläche befinden.

Die Halterung 30 für das Tauchrohr kann sowohl fest mit dem Behälter oder wie dargestellt mit dem Einbauteil oder aber mit dem Ventilgehäuse verbunden sein. In allen Fällen bleibt das Tauchrohr relativ zum Behälter ortsfest.

Je nach Einzelfall kann aber auch eine Lösung, wie in Fig. 3 dargestellt, gewählt werden, bei der das Tauchrohr 31 fest mit dem Ventilstößel verbunden ist und sich somit mit dem Ventilstößel relativ zum Behälter bewegt. Die Bewegung kann auch kraftbetätigt durch Zylinder oder elektrische Linearantriebe erfolgen, die mit der Ventilspindel 8 verbunden sind.

Für spezielle Einsatzfälle, bei denen die auf der Ventilspindel 8 gleitende Dichtung 25 ersetzt werden muß, um eine höhere Sicherheit gegenüber Kontamination zu erreichen, kann diese gleitende Dichtung auch durch eine Membrandichtung ersetzt sein, die auf der einen Seite fest in dem Ventilgehäuse 5 gehalten ist und auf der anderen Seite mit der Ventilspindel 8 verbunden ist. Damit der notwendige Hub der Ventilspindel 8 gewährleistet wird, kann eine derartige Dichtung elastisch, entweder als Faltenbalg oder als Schlauch ausgebildet sein.

Für pathogene Anwendungen sind auch Doppeldichtungen mit drucküberlagerten Sperräumen ausführbar. Als Sperrmedium dient zum Beispiel Dampfkondensat, das unter genügenden Überdruck gesetzt ist, so daß bei Undichtigkeiten kein Arbeitsmedium austreten kann.

## Patentansprüche

1. Sterilisierbares Objekt, wie Behälter oder Leitung, mit Anschlußventil, das eine äußere Anschlußöffnung (10), ein an einer Anschlußöffnung (3) des Objekts (2) dichtend anbringbares Ventilgehäuse (5) mit zum Innenraum (6) des Objekts (2) führender Sitzöffnung (7), eine in dieser zwischen einer Sperrstellung (A) und einer Durchtrittsstellung (B) radial abgedichtet verschiebbar geführte Ventilspindel (8) aufweist, **dadurch gekennzeichnet,** daß im Ventilgehäuse (5) eine an die Umfangsfläche der Ventilspindel (8) angrenzende Ventilkammer (9) angeordnet ist, die mit der außenseitigen Anschlußöffnung (10) in Verbindung steht, und die Ventilspindel (8) endseitig eine Durchgangsbohrung (11) mit mindestens einer in vorbestimmtem Abstand vom Ende der Ventilspindel in deren Umfangsfläche mündende Seitenöffnung (12) aufweist, die in der Sperrstellung (A) permanent mit dem Innenraum (6) des Behälters (2) kommuniziert, wobei alle Öffnungen (12) der Durchgangsbohrung (11) im Innenraum (6) des Behälters (2) liegen, und in der Durchtrittsstellung (B) die Durchgangsbohrung (11) über die Seitenöffnungen (12) mit der Ventilkammer kommuniziert (Fig. 1 oder Fig. 4).

2. Sterilisierbares Objekt, wie Behälter oder Leitung, mit Anschlußventil, das eine äußere Anschlußöffnung (10), ein an einer Anschlußöffnung (3) des Objekts (2) dichtend anbringbares Ventilgehäuse (5) mit zum Inneraum (6) des Objekts (2) führender Sitzöffnung (7), eine in dieser zwischen einer Sperrstellung (A) und einer Durchtrittsstellung (B) radial abgedichtet verschiebbar geführte Ventilspindel (8) aufweist, **dadurch gekennzeichnet,** daß die Ventilspindel (8) eine als Anschlußöffnung (10) ausgebildete Durchgangsbohrung aufweist, die in mindestens eine Seitenöffnung (12) (Fig. 5) mündet.

3. Sterilisierbares Objekt, wie z.B. Behälter oder Leitung, mit Anschlußventil, das eine äußere Anschlußöffnung (10), ein an einer Anschlußöffnung (3) des Behälters (2) dichtend anbringbares Ventilgehäuse (5) mit zum Innenraum (6) des Objekts (2) führender Sitzöffnung (7), eine in dieser zwischen einer Sperrstellung (A) und einer Durchtrittsstellung (B) radial abgedichtet verschiebbar geführte Ventilspindel (8) aufweist, **dadurch gekennzeichnet,** daß im Ventilgehäuse (5) eine an die Umfangsfläche der Ventilspindel (8) angrenzende Ventilkammer (9) angeordnet ist, die mit der außenseitigen Anschlußöffnung (10) in Verbidnung steht, und die Ventilspindel (8) eine Durchgangsbohrung (11) (Fig. 6) aufweist, die an ihren beiden Enden auf der Umfangsfläche der Ventilspindel (8) Seitenöffnungen (12) aufweist, wobei in Sperrstellung (A) alle Öffnungen (12) der Durchgangsbohrung (11) im Innenraum des Behälters oder in der Ventilkammer liegen, und in der Durchtrittsstellung (B) ein Teil der Öffnungen mit der Ventilkammer und der andere Teil der Öffnungen der Durchgangsbohrungen mit dem Behälter kommuniziert.

4. Sterilisierbares Objekt nach Anspruch 1, 2 oder 3, **dadurch gekennzeichnet,** daß das Anschlußventil eine Verbindung zu einem Tauchrohr (31) aufweist.

5. Sterilisierbares Objekt nach Anspruch 1, 2, 3 oder 4, **dadurch gekennzeichnet,** daß das von dem Behälterinnenraum (6) abgewandte Ende der Ventilspindel (8) an mindestens zwei Stellen (25,26) gegenüber dem Ventilgehäuse (5) abgedichtet ist, so daß die Dichtungen einen Sperraum (16) um die Spindel (8) bilden, in den eine oder mehrere Bohrungen (17,18) mündend angeordnet sind.

6. Sterilisierbares Objekt nach Anspruch 1, 2, 3, 4 oder 5, **dadurch gekennzeich-net,** daß das Anschlußventil eine Verbindung zu einem in Richtung des Behälters (2) öffnenden Rückschlagventil aufweist.

7. Sterilisierbares Objekt nach Anspruch 1, 2, 3, 4, 5 oder 6, **dadurch gekennzeich-net,** daß das Ventilgehäuse (5) innerhalb eines Einbauteils (13) angeordnet ist.

8. Sterilisierbares Objekt nach Anspruch 7, **da-durch gekennzeichnet,** daß die Ventilspindel (8), das Ventilgehäuse (5) und das Einbauteil (13) die Ventilkammer (9) bildend angeordnet sind (Fig. 8).

9. Sterilisierbares Objekt nach Anspruch 5, 6, 7 oder 8, **dadurch gekennzeichnet,** daß die Dichtung (25) zwischen Ventilspindel (8) und Ventilgehäuse (5) auf der dem Behälterinneraum (16) abgewandten Seite eine Membrandichtung ist.

## Claims

1. A sterilisable object such as a container or duct with a connecting valve comprising an outer connecting opening (10), a valve casing (5) for attaching in sealing-tight manner to a connecting opening (3) on the object (2) and with a seat opening (7) leading to the interior (6) of the object (2), and a radially sealed valve stem (8) guided for movement in the seat opening (7) between a shut position (A) and an open position (B), characterised in that a valve chamber (9) adjacent the peripheral surface of the valve stem (8) is disposed in the casing and connected to the outer connecting opening (10), and the valve stem (8) ends in a through bore (11) having at least one side opening (12) at a predetermined distance from the end of the stem and terminating in the peripheral surface thereof and, in the shut position (A), permanently communicating with the interior (6) of the container (2), all openings (12) of the through bore (11) being situated in the interior (6) of the chamber (2), and the through bore (11) communicating via the side openings (12) with the valve chamber when in the open position (B) (Fig. 1 or Fig. 4).

2. A sterilisable object such as a container or duct with a connecting valve comprising an outer connecting opening (10), a valve casing (5) for attaching in sealing-tight manner to a connecting opening (3) on the object (2) and with a seat opening (7) leading to the interior (6) of the object (2), and a radially sealed valve stem (8) guided for movement in the seat opening (7) between a shut position (A) and an open position (B), characterised in that the valve stem (8) has a through bore in the form of a connecting opening (10) terminating in at least one side opening (12) (Fig. 5).

3. A sterilisable object such as a container or duct with a connecting valve comprising an outer connecting opening (10), a valve casing (5) for attaching in sealing-tight manner to a connecting opening (3) on the container (2) and with a seat opening (7) leading to the interior (6) of the object (2), and a radially sealed valve stem (8) guided for movement in the seat opening (7) between a shut position (A) and an open position (B), characterised in that a valve chamber (9) adjacent the peripheral surface of the valve stem (8) is disposed in the casing (5) and connected to the outer connecting opening (10), and the valve stem (8) has a through bore (11) (Fig. 6), formed at both ends with side openings (12) on the peripheral surface of the valve stem (8), and in the shut position (A) all the openings (12) of the through bore (11) are situated in the interior of the container or in the valve chamber, whereas in the open position (B), some of the openings of the through bores communicate with the valve chamber and the rest of the openings communicate with the container.

4. A sterilisable object according to claim 1, 2 or 3, characterised in that the connecting valve has a connection to an immersion tube (31).

5. A sterilisable object according to claim 1, 2, 3 or 4, characterised in that the end of the valve stem (8) remote from the interior (6) of the container is sealed against the valve casing (5) at at least two places (25, 26), so that the seals form a barrier chamber (16) around the stem (8), into which one or more bores (17, 18) terminate.

6. A sterilisable object according to claim 1, 2, 3, 4 or 5, characterised in that the connecting valve has a connection to a non-return valve opening in the direction of the container (2).

7. A sterilisable object according to claim 1, 2, 3, 4, 5 or 6, characterised in that the valve casing (5) is disposed inside an insertion part (13).

8. A sterilisable object according to claim 7, characterised in that the valve spindle (8), the valve casing (5) and the insertion part (13) are disposed so as to form the valve chamber (9) (Fig. 8).

9. A sterilisable object according to claim 5, 6, 7 or 8, characterised in that the seal (25) between the valve stem (8) and the valve casing (5) on the side remote from the interior (16) of the container is a diaphragm seal.

## Revendications

1. Objet stérilisable, comme un récipient ou une tubulure, avec une vanne de connexion qui comporte une ouverture de connexion extérieure (10), une cage de vanne (5) applicable, en assurant l'étanchéité, à une ouverture de connexion (3) de l'objet (2), avec une ouverture de siège (7) conduisant vers l'intérieur (6) de l'objet (2), une tige de vanne (8) guidée en coulissement dans celle-ci en assurant l'étanchéité radialement entre une position d'arrêt (A) et une position de passage (B), caractérisé en ce que dans la cage de soupape (5) est placée une chambre de soupape (9) contiguë à la surface périphérique de la tige de vanne (8), chambre qui est en communication avec l'ouverture de connexion (10) du côté extérieur, et la tige de vanne (8) comporte, du côté de l'extrémité, un alésage de passage (11) avec au moins une ouverture latérale (12) débouchant dans sa surface périphérique à une distance prédéterminée de l'extrémité de la tige de vanne, ouverture qui communique, en position d'arrêt A, de manière permanente avec l'intérieur (6) du récipient (2), toutes les ouvertures (12) se situant à l'intérieur du récipient (2) et, dans la position de passage (B), l'alésage de passage (11) communiquant par les ouvertures latérales (12) avec la chambre de soupape (figure 1 ou figure 4).

2. Objet stérilisable, comme un récipient ou une tubulure, avec une vanne de construction qui comporte une ouverture de connexion (10), une cage de vanne (5) applicable à une ouverture de raccordement (3) de l'objet (2) en réalisant l'étanchéité avec une ouverture de siège (7) conduisant vers l'intérieur (6) de l'objet (2), une tige de vanne (8) guidée en coulissement dans celle-ci en assurant l'étanchéité entre une position d'arrêt (A) et une position de passage (B), caractérisée en ce que la tige de vanne (8) comprend un alésage de passage réalisé comme ouverture de raccordement (10), qui débouche dans au moins une ouverture latérale (12) (figure 5).

3. Objet stérilisable, comme un récipient ou une tubulure avec une vanne de connexion qui comporte une ouverture de connexion extérieure (10), une cage de vanne (5) applicable, en assurant l'étanchéité, à une ouverture de connexion (3) de l'objet (2), avec une ouverture de siège (7) conduisant vers l'intérieur (6) de l'objet (2), une tige de vanne (8) guidée en coulissement dans celle-ci en assurant l'étanchéité radialement entre une position d'arrêt (A) et une position de passage (B), caractérisé en ce que dans la cage de soupape (5) est disposée une chambre de soupape (9) contiguë à la surface périphérique de la tige de soupape (8), chambre qui est en communication avec l'ouverture de connexion (10) du côté extérieur, et la tige de vanne (8) comprend un alésage de passage (11) (figure 6) qui a à ses deux extrémités des ouvertures latérales (12) sur la surface périphérique de la tige de vanne (8), toutes les ouvertures (12) de l'alésage de passage (11) étant situées en position d'arrêt (A) à l'intérieur du récipient ou dans la chambre de soupape, et une partie des ouvertures communiquant en position de passage (B) avec la chambre de soupape, et l'autre partie des ouvertures des alésages de passage communiquant avec le récipient.

4. Objet stérilisable selon les revendications 1, 2 ou 3, caractérisé en ce que la vanne de connexion est en liaison avec un tube plongeur (31).

5. Objet stérilisable selon les revendications 1, 2, 3, ou 4, caractérisé en ce que l'extrémité de la tige de vanne (8) éloignée de l'intérieur du récipient (6) est rendue étanche par rapport à la cage de vanne (5) au moins en deux endroits (25, 26), de sorte que les garnitures d'étanchéité constituent un espace d'arrêt (16) autour de la tige, espace dans lequel sont disposés et débouchent un ou plusieurs perçages (17, 18).

6. Objet stérilisable selon les revendications 1, 2, 3,4 ou 5, caractérisé en ce que la vanne de connexion comporte une liaison avec une soupape anti-retour s'ouvrant en direction du récipient (2).

7. Objet stérilisable selon les revendications 1, 2, 3, 4, 5 ou 6, caractérisé en ce que la tige de vanne (8), la cage de vanne (5) et la pièce de montage (13) sont disposées en formant la chambre de vanne (9). (figure 7)

8. Objet stérilisable selon la revendication 7, caractérisé en ce que la tige de vanne (8), la cage de vanne (5) et la pièce de montage (13) sont disposées en formant la chambre de vanne (9). (figure 8)

9. Objet stérilisable selon la revendication 8, caractérisé en ce que la garniture d'étanchéité (25) est une garniture à membrane entre la tige de vanne (8) et la cage de vanne (5) sur le côté éloigné de l'intérieur du récipient (16).
